# FASCICULE DE BREVET EUROPEEN

(11) **EP 3 649 107 B1**
(45) Date de publication et mention de la délivrance du brevet: **12.10.2022**
(21) Numéro de dépôt: 18734580.6
(22) Date de dépôt: 04.07.2018
(51) Int. Cl.: C07C 311/07, H01M 6/00

(54) **MACROMOLÉCULES DE TYPE SULFONAMIDE UTILES À TITRE D'ÉLECTROLYTE POLYMÈRE À CONDUCTION D'ION UNIQUE**
ALS EINZELIONEN-LEITENDER POLYMERELEKTROLYT NÜTZLICHE SULFONAMID-MAKROMOLEKÜLE
SULFONAMIDE MACROMOLECULES USEFUL AS SINGLE-ION CONDUCTING POLYMER ELECTROLYTE

(30) Priorité: 04.07.2017 FR 1756291
(43) Date de publication de la demande: 13.05.2020
(73) Titulaire: Commissariat à l'Énergie Atomique et aux Énergies Alternatives, 75015 Paris (FR)
(72) Inventeur: OVERTON, Philip, 38000 Grenoble (FR); PICARD, Lionel, 38170 Seyssinet-Pariset (FR); RANNOU, Patrice, 38320 Eybens (FR)
(74) Mandataire: Nony
(86) Numéro de dépôt international: PCT/EP2018/068135
(87) Numéro de publication internationale: WO 2019/008061

(56) Documents cités:
- YAPIN WANG ET AL: "A Convenient Reductive Deamination (Hydrodeamination) of Aromatic Amines", THE JOURNAL OF ORGANIC CHEMISTRY, vol. 66, no. 25, 1 décembre 2001 (2001-12-01), pages 8293-8296, XP055445657, US ISSN: 0022-3263, DOI: 10.1021/jo010681w
- AELONY D: "SOME SULFONAMIDE PLASTICIZERS AND WAXES", INDUSTRIAL AND ENGINEERING CHEMIS, AMERICAN CHEMICAL SOCIETY, US, vol. 46, 1 janvier 1954 (1954-01-01), pages 587-591, XP008000300, DOI: 10.1021/IE50531A049
- M. M. DUGUET: "Sur les écarts constatés dans les poits de fusion des quelques nouvelles amides dérivant des acides sulfoniques aliphatiques", RECUEIL DES TRAVAUX CHIMIQUES DES PAYS-BAS ET DE LA BELGIQUE, vol. 25, 1 janvier 1906 (1906-01-01), pages 213-228, XP009503144,
- RENAUD BOUCHET ET AL: "Single-ion BAB triblock copolymers as highly efficient electrolytes for lithium-metal batteries", NATURE MATERIALS, vol. 12, no. 5, 31 mars 2013 (2013-03-31), pages 452-457, XP055316945, GB ISSN: 1476-1122, DOI: 10.1038/nmat3602 cité dans la demande

## Description

La présente invention se rapporte au domaine des électrolytes polymères conducteurs d'ion unique et vise notamment à proposer de nouveaux sels polymères asymétriques pour le transport des cations, tel que le lithium, dans des systèmes électrochimiques.

Les principaux systèmes électrochimiques utilisent le cation lithium comme espèce ionique de transport. Par exemple, dans le cas d'un accumulateur au lithium, le cation lithium extrait de la cathode lors de la charge de la batterie vient se déposer sur l'anode, et inversement, il s'extrait de l'anode pour s'intercaler dans la cathode lors de la décharge. Son transport est assuré par un électrolyte conducteur ionique.

La nature de l'électrolyte conducteur ionique utilisé revêt un caractère essentiel pour les performances du système électrochimique. La conductivité ionique de l'électrolyte conditionne notamment l'efficacité du système électrochimique étant donné qu'elle intervient sur la mobilité des ions entre les électrodes positive et négative.

D'autres paramètres interviennent également dans le choix de l'électrolyte mis en œuvre. Il s'agit notamment de sa stabilité thermique, chimique ou électrochimique au sein du système électrochimique, ainsi que des critères économiques, de sécurité et de respect de l'environnement.

D'une manière générale, les électrolytes des systèmes électrochimiques se présentent sous forme liquide, gélifiée ou solide.

La présente invention s'intéresse plus particulièrement aux électrolytes sous forme solide, et plus particulièrement aux électrolytes polymères solides, qui se révèlent plus sûrs sur le plan de la sécurité et ne nécessitent pas obligatoirement l'utilisation d'un séparateur.

A titre illustratif de ces électrolytes polymères peuvent notamment être cités des poly(oxyéthylène) POE dans lesquels est classiquement dissous un sel de métal alcalin ou alcalino-terreux suivant la chimie des électrodes. Toutefois, ces électrolytes présentent des performances limitées en matière de conductivité ionique liées au mécanisme de transport du cation, dit « assisté », et nécessitent une température d'utilisation élevée (60 °C à 80 °C).

Différentes études ont donc été menées en vue d'augmenter les performances de conduction ionique des électrolytes polymères.

Il a ainsi été proposé un mélange d'un polystyrène porteur de groupements sulfonyl(trifluorométhylsulfonyl)imide et de POE pour réaliser une membrane d'électrolyte (Meziane et al. Electrochimica Acta, 2011, 57, 14-19). Ces électrolytes polymères présentent néanmoins des conductivités ioniques insuffisantes, de l'ordre de 9,5.10⁻⁶ S/cm à 70 °C.

Il a également été considéré, pour les systèmes d'électrolyte au lithium, l'incorporation d'un sel de lithium bis (trifluorométhane) sulfonimide (LiTFSI) dans l'unité répétitive, conjointement au styrène, pour former du poly(styrène trifluorométhanesulfonylimide de lithium) P(STFSILi). A l'issue de la polymérisation, le poly(électrolyte) possède une architecture copolymère bloc BAB comportant un bloc central «A» de PEO. La conductivité maximale, de l'ordre de 10⁻⁵ S/cm, est obtenue à 60 °C avec un polymère comportant 78 % en masse de POE. (R Bouchet et al., Nature Materials (2013), 12, 452). L'incorporation d'un groupe fonctionnel en extrémité d'un copolymère à blocs de ce type s'est révélée bénéfique pour sa conductivité. (G. Lo et al., ACS Macro Lett. (2013), 2, 990 et H. Jung et al., Macromolecules (2017), 50, 3224)

Pour des raisons évidentes, l'amélioration des performances des électrolytes relève d'un objectif permanent.

Il demeure donc un besoin de disposer d'un électrolyte possédant une conductivité ionique élevée et possédant de préférence un nombre de transport du cation le plus proche de l'unité possible.

Il demeure également un besoin de disposer d'un électrolyte possédant une stabilité électrochimique améliorée.

La présente invention vise précisément à proposer de nouveaux composés utiles à titre d'électrolytes, conducteurs ioniques, cationiques ou protoniques car présentant une conductivité ionique ou protonique et une stabilité électrochimique améliorées.

Plus particulièrement, elle concerne, selon un premier de ses aspects, un composé de type sulfonamide asymétrique, notamment utile à titre d'électrolyte polymère à conduction d'ion unique, comprenant au moins :
- un groupement polycyclique, Ar, formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ;
- une chaîne aliphatique, linéaire ou ramifiée, saturée ou insaturée, ladite chaîne étant éventuellement interrompue par un ou plusieurs hétéroatomes, par un ou plusieurs métalloïdes, éventuellement substituée par un ou plusieurs atomes de fluor, et/ou contenant un ou plusieurs groupes choisis dans le groupe constitué des groupes hydroxyle, -NH₂ et oxo ;
- ledit groupement Ar et ladite chaine aliphatique étant liés de manière covalente *via* un espaceur figuré par un motif sulfonamide -SO₂-NH-, ou sa forme anionique -SO₂-N⁻- ; et, le cas échéant
- un contre-cation de la forme anionique du motif sulfonamide, choisi parmi les métaux alcalins et le proton H⁺ ou H₃O⁺.

En particulier, les composés selon l'invention sont sous la forme d'un sel possédant à titre d'espaceur, la forme anionique du sulfonamide -SO₂-N⁻-, et un contre-cation, de préférence alcalin, et en particulier un atome de lithium.

En particulier, le motif sulfonamide est lié audit groupement polycyclique *via* son atome d'azote et à ladite chaîne aliphatique *via* son atome de soufre.

Plus particulièrement, les composés de type sulfonamide asymétrique selon l'invention comprennent au moins :
- un groupement polycyclique, Ar, formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ;
- une chaîne aliphatique, linéaire ou ramifiée, saturée ou insaturée, ladite chaîne étant éventuellement interrompue par un ou plusieurs hétéroatomes, par un ou plusieurs métalloïdes, éventuellement substituée par un ou plusieurs atomes de fluor, et/ou contenant un ou plusieurs groupes choisis dans le groupe constitué des groupes hydroxyle, -NH₂ et oxo, ladite chaîne aliphatique comprenant un enchaînement linéaire d'au moins six liaisons covalentes ;
- ledit groupement Ar et ladite chaine aliphatique étant liés de manière covalente *via* un espaceur figuré par un motif sulfonamide -SO₂-NH- ou sa forme anionique
   -SO₂-N⁻-, ledit sulfonamide étant lié audit groupement polycyclique Ar *via* son atome d'azote et à ladite chaîne aliphatique *via* son atome de soufre ; et, le cas échéant
- un contre-cation de la forme anionique du motif sulfonamide, choisi parmi les métaux alcalins et le proton H⁺ ou H₃O⁺.

En particulier, le lien covalent entre l'atome d'azote et ledit groupement polycyclique est établi *via* une structure aromatique formant tout ou partie dudit groupement polycyclique.

En particulier, le groupement polycyclique Ar est sous une forme condensée aromatique.

Certes, des sulfonamides ont déjà été considérés pour l'élaboration de (co)-solvants pour les batteries lithium-ion. Toutefois, l'architecture des composés correspondants est très différente. Les composés possèdent pour l'essentiel une liaison de l'atome de soufre du sulfonamide à des chaînes alkyles fluorées courtes et de l'azote de ce sulfonamide à des fonctions hydrocarbonées simples. (D Bresser et al. EP 3 050 872)

Les sulfonamides liés à une espèce aromatique sont rares dans la littérature publiée. A la connaissance des inventeurs, seule la publication H. Ohno et al. (Solid State Ionics (1999), 124, 323) décrit des composés dont le motif sulfonamide est lié *via* son atome d'azote à un phényle. Ce sel asymétrique peut être en outre connecté *via* l'atome de soufre de l'anion sulfonamide à un polymère de type PEO (550 g/mol). En conséquence, les composés décrits dans ce document sont dénués du motif polycyclique considéré selon l'invention.

Or, les composés selon l'invention, lorsqu'utilisés à titre d'électrolytes, s'avèrent particulièrement intéressants au regard de leur spécificité originale de posséder un tel groupement polycyclique.

Comme il ressort de ce qui précède, l'atome d'azote du motif sulfonamide anionique est lié par covalence à un groupe aromatique (Ar). Pour sa part, l'atome de soufre du motif sulfonamide est lié à un groupe de type oligomère ou polymère. C'est la déprotonation du motif sulfonamide qui génère la base de Lewis. Or, avantageusement, le caractère base de Lewis du sulfonamide est augmenté par la structure aromatique, à laquelle il est lié *via* son atome d'azote. En effet, la charge négative de la base de Lewis se trouve délocalisée sur les atomes d'oxygène du sulfonamide, par rapport aux électrons π délocalisés au niveau de la structure aromatique. Ainsi, la structure aromatique permet d'augmenter les caractéristiques physico-chimiques du sel asymétrique de deux manières.

Tout d'abord, elle contribue à la stabilisation électronique du sel par délocalisation de sa charge anionique. La structure aromatique, localisée à proximité, car liée à l'atome d'azote du sulfonamide, renforce la densité électronique anionique et cette délocalisation améliore ainsi la dissociation du cation, généralement le lithium, du composant anionique du composé selon l'invention.

Par ailleurs, la structure aromatique contribue par sa taille, en tant qu'agent structurant directionnel, à l'organisation structurale du polymère à l'état solide. Un motif aromatique monocyclique, de taille plus réduite à l'image d'un radical phényle, est moins capable de diriger l'organisation structurale du sel qu'un motif polycyclique de taille plus importante. Les interactions connues d'empilement π-π (Van der Waals) des anneaux aromatiques proximaux ont un effet significatif pour diriger l'organisation de la structure, en combinaison avec le volume stérique de la structure aromatique polycyclique. Un groupement naphtalène fonctionnalisé est connu pour s'auto-assembler *via* l'empilement π-π (interaction de Van der Waals) et les dimensions d'un cycle aromatique plus grand, en particulier par rapport au benzène, introduisent une dimension stérique favorable à cet auto-assemblage. Les inventeurs ont ainsi constaté que la taille et la position du groupement aromatique Ar dans l'architecture macromoléculaire des composés selon l'invention sont des paramètres déterminants pour l'efficacité des électrolytes qui en dérivent.

Avantageusement, la conductivité ionique d'un électrolyte selon l'invention est basée sur un mécanisme de conduction « direct », par « saut » (« hopping » en langue anglaise) des cations C^{x+} du groupement sulfonamide anionique, localisé sur un groupement polycyclique Ar, à l'autre.

Selon un autre de ses aspects, la présente invention se rapporte à l'utilisation de la forme anionique d'un sulfonamide asymétrique selon l'invention à titre d'électrolyte.

Selon encore un autre de ses aspects, la présente invention se rapporte à un électrolyte formé en tout ou partie par la forme anionique d'un sulfonamide asymétrique selon l'invention.

En particulier, la forme anionique considérée est un sel alcalin, et en particulier le sel de lithium.

Les macromolécules selon l'invention peuvent être mises en œuvre comme électrolytes dans de nombreux systèmes électrochimiques, tels que les générateurs, par exemple les batteries au lithium, et les systèmes de conversion électrochimique, par exemple les piles à combustible à membrane échangeuse de protons (PEMFC).

D'autres caractéristiques, variantes et avantages des molécules et électrolytes selon l'invention, de leur préparation et de leur mise en œuvre ressortiront mieux à la lecture de la description, des exemples et figures qui vont suivre, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

### COMPOSES DE TYPE SULFONAMIDES DE L'INVENTION

Comme énoncé précédemment, les composés de type sulfonamide asymétrique selon l'invention comprennent au moins :
- un groupement polycyclique, Ar, formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ;
- une chaîne aliphatique, linéaire ou ramifiée, saturée ou insaturée, ladite chaîne étant éventuellement interrompue par un ou plusieurs hétéroatomes, par un ou plusieurs métalloïdes, notamment contenant un atome de silicium, éventuellement substituée par un ou plusieurs atomes de fluor, et/ou contenant un ou plusieurs groupes choisis dans le groupe constitué des groupes hydroxyle, -NH₂ et oxo, ladite chaîne aliphatique comprenant en particulier un enchaînement linéaire d'au moins six liaisons covalentes ;
- ledit groupement Ar et ladite chaine aliphatique étant liés de manière covalente *via* un espaceur figuré par un motif sulfonamide -SO₂-NH-, ou sa forme anionique
   -SO₂-N⁻- ; ledit sulfonamide étant notamment lié audit groupement polycyclique Ar *via* son atome d'azote et à ladite chaîne aliphatique *via* son atome de soufre ; et, le cas échéant
- un contre-cation choisi parmi les métaux alcalins et le proton H⁺ ou H₃O⁺.

### Groupement polycyclique

Dans le cadre de l'invention, on entend par « groupement polycyclique », un groupement présentant deux ou plusieurs noyaux (cycles), condensés (ortho-condensés ou ortho- et péri-condensés) les uns aux autres, c'est-à-dire présentant, deux à deux, au moins deux carbones en commun.

En particulier, un groupement polycyclique selon l'invention est formé de deux à six cycles, les cycles comprenant, indépendamment les uns des autres, de 4 à 6 chaînons. Le groupement polycyclique est dit aromatique dans la mesure où il comprend au moins un cycle aromatique qui est notamment impliqué dans la liaison covalente avec l'atome d'azote du motif sulfonamide.

Ainsi, le groupement polycyclique peut être en partie aromatique ou totalement aromatique.

Le groupement polycyclique peut inclure un ou plusieurs hétéroatomes. On parle alors de « groupement polyhétérocyclique ».

On entend par « (hétéro)cycle ou non de 4 à 6 chaînons », un groupe cyclique insaturé, partiellement saturé ou saturé, à 4, 5 ou 6 chaînons, comprenant éventuellement un ou plusieurs hétéroatomes, en particulier choisis dans le groupe constitué de l'oxygène, le soufre et l'azote.

En particulier, le groupement polycyclique dit Ar est un groupement polycyclique aromatique formé de 2 à 6 cycles aromatiques fusionnés à 6 chaînons, de préférence de 2 à 6 cycles benzéniques fusionnés, avantageusement de 2 à 4 cycles benzéniques fusionnés.

Plus particulièrement, Ar peut présenter l'un des squelettes polycycliques suivants ou dériver de l'un des squelettes polycycliques suivants :

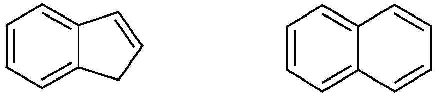

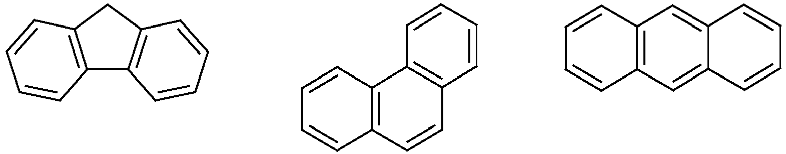

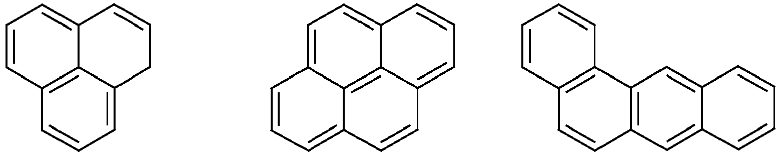

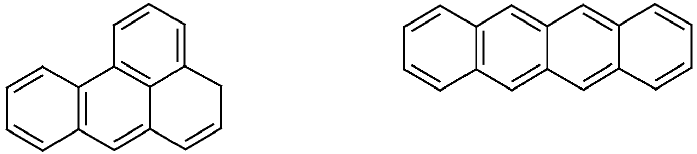

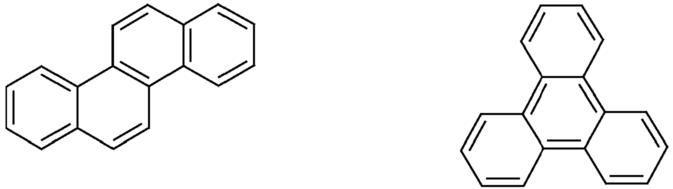

Comme énoncé précédemment, il est entendu que le groupement Ar peut être un groupement polyhétérocyclique c'est-à-dire présentant l'un des squelettes présentés ci-dessus mais dans lesquels un ou plusieurs atomes de carbone sont remplacés par un ou plusieurs hétéroatomes, notamment choisis dans le groupe constitué de S, N et O.

Selon un mode de réalisation particulier, Ar est un radical dérivant d'un squelette bicyclique aromatique, et plus particulièrement un radical du naphtalène ou dit encore radical naphtyle.

### Chaîne aliphatique

Au sens de l'invention une chaîne aliphatique, linéaire ou ramifiée, saturée ou insaturée peut posséder une structure d'oligomère, d'homopolymère, de copolymère ou non.

Un polymère peut en particulier être caractérisé *via* un nombre d'unité de répétition (DPn). En particulier, il peut posséder un DPn supérieur à 5.

Selon un mode de réalisation, la chaîne aliphatique comprend un enchaînement linéaire d'au moins 6 liaisons covalentes.

Selon un mode de réalisation particulier, elle peut est formée d'un unique segment ou d'un enchaînement linéaire d'au moins deux segments de chaîne, en particulier de deux ou trois segments de chaîne de nature chimique différente.

De préférence, elle consiste en une chaîne alkyle linéaire comprenant de 6 à 18 atomes de carbone, de préférence de 6 à 12 atomes de carbone, éventuellement fluorée, semifluorée (c'est-à-dire avec une alternance de motifs méthylène et perfluorométhylène), voire perfluorée, et/ou éventuellement substituée par au moins un, voire plusieurs groupe(s) hydroxyle(s), et éventuellement entrecoupée par un ou plusieurs atome(s) d'oxygène.

En particulier, la chaîne aliphatique est un radical octyle.

En particulier, la chaîne aliphatique possède un enchainement d'au moins 5, voire plus, motifs oxyde d'éthylène (OE).

### Motif sulfonamide

Dans les composés sulfonamide asymétriques selon l'invention, le motif sulfonamide peut se présenter sous forme -SO₂-NH- ou -SO₂-N⁻-.

De préférence, il s'agit de la forme anionique -SO₂-N⁻- et le composé selon l'invention comprend en outre un cation, choisi parmi le proton H⁺ ou H₃O⁺ (proton hydraté)et les métaux alcalins. Lorsque le cation est choisi dans le groupe des métaux alcalins, le composé selon l'invention est alors qualifié de sel.

Selon une variante de réalisation, l'atome d'azote du motif anionique sulfonamide est lié de manière covalente à un atome de carbone ou un hétéroatome du groupement polycyclique Ar.

Comme détaillé dans la suite du texte, de telles macromolécules peuvent être avantageusement mises en œuvre à titre d'électrolyte dans une batterie au lithium.

### Métaux alcalins

Au sens de l'invention le terme « métaux alcalins », désigne les éléments chimiques de la première colonne du tableau périodique des éléments, et plus particulièrement choisis dans le groupe constitué du lithium, du sodium, du potassium, du rubidium, et du césium.

De préférence, le métal alcalin est le lithium, le sodium ou le potassium, et plus préférentiellement le lithium.

Selon une autre variante de réalisation de l'invention, le contre-cation représente le proton H⁺ ou H₃O⁺, et l'atome de soufre est lié de manière covalente à un atome de carbone ou un hétéroatome du groupement polycyclique Ar. De telles macromolécules peuvent être avantageusement mises en œuvre à titre d'électrolyte dans une pile à combustible à membrane échangeuse de protons (PEMFC) un électrolyseur basse température, ou une batterie redox-flow.

Selon un mode de réalisation particulier, les molécules selon l'invention répondent à la formule (I) suivante : 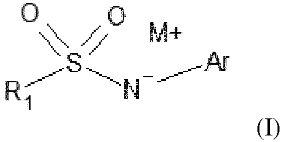 dans laquelle
- Ar est un groupement polycyclique totalement aromatique tel que défini précédemment et de préférence représente un radical naphtyle,
- R₁ représente une chaîne aliphatique telle que définie précédemment et
- M⁺ est un contre-cation choisi dans le groupe des métaux alcalins et en particulier est le Li⁺.

En particulier, R₁ est une chaîne alkyle linéaire, qui comprend de 4 à 18 atomes de carbone, de préférence de 6 à 16 atomes de carbone, éventuellement substituée par un ou plusieurs groupe(s) hydroxyle(s), et/ou éventuellement entrecoupée par un ou plusieurs atome(s) d'oxygène.

Comme énoncé précédemment, la chaîne alkyle linéaire peut posséder un enchainement d'au moins 5, voire plus, motifs OE.

A titre représentatif et non limitatif des composés de type sulfonamide selon l'invention, peuvent notamment être cités les composés suivants :

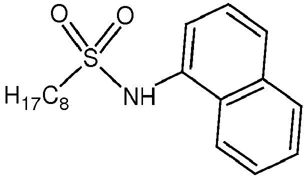

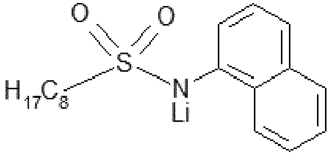

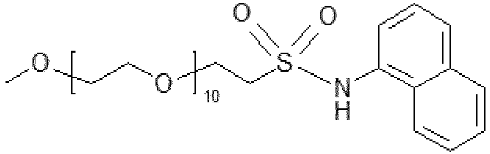

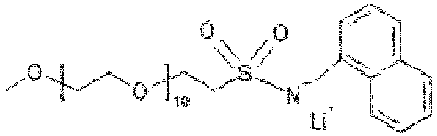

### Préparation des composés de l'invention

Les macromolécules selon l'invention peuvent être obtenues selon des voies de synthèse relevant clairement des compétences de l'homme du métier.

En particulier, certaines peuvent être préparées en mettant en œuvre des méthodes de substitution ou d'addition nucléophile connues de l'homme du métier, comme détaillé ci-dessous. Ce type de réactions met en présence un précurseur du groupement polycylique, Ar, et un précurseur de la chaîne aliphatique.

Selon un mode de réalisation, le précurseur de Ar, est porteur d'un groupe nucléophile, par exemple de type amine, hydroxyle ou thiol et de préférence amine, et le précurseur de la chaîne aliphatique est porteur d'un groupe électrophile de type chlorure de sulfonyle.

La formation, au préalable, de chacun des précurseurs peut être également à considérer.

A titre illustratif des méthodes que l'homme du métier peut employer pour parvenir aux molécules de l'invention, une méthode de préparation des molécules de l'invention de formule (I) est décrite ci-après et également dans les exemples qui suivent.

Bien entendu, il appartient à l'homme du métier d'ajuster les conditions de synthèse pour obtenir les macromolécules selon l'invention.

### UTILISATION DES COMPOSES SULFONAMIDE SELON L'INVENTION COMME ELECTROLYTE

Selon un autre de ses aspects, la présente invention se rapporte à un électrolyte comprenant, au moins un, voire étant formé d'un sel de sulfonamide asymétrique tel que défini ci-dessus.

L'électrolyte selon l'invention présente de bonnes propriétés de conductivité ionique.

De préférence, un électrolyte de l'invention présente une conductivité ionique à 20 °C supérieure ou égale à 10⁻⁸ S/cm, en particulier comprise entre 10⁻⁷ S/cm et 10⁻³ S/cm et une conductivité ionique à 150 °C supérieure ou égale à 10⁻⁵ S/cm.

La conductivité ionique peut être mesurée par spectroscopie d'impédance électrochimique en tension ou en courant, d'après une méthode connue de l'homme du métier.

L'électrolyte selon l'invention peut être mis en œuvre dans un système électrochimique, par exemple une batterie au lithium.

La présente invention concerne ainsi, selon encore un autre de ses aspects, un système électrochimique comprenant un électrolyte selon l'invention.

Dans le système électrochimique de l'invention, l'électrolyte est de préférence imprégné sur un séparateur poreux tel que décrit précédemment.

Le système électrochimique peut être un système générateur, convertisseur ou de stockage électrochimique.

Il peut s'agir plus particulièrement d'une pile à combustible, par exemple une pile à combustible à membrane échangeuse de protons (PEMFC) ou batterie redox-flow; une batterie primaire ou secondaire, par exemple une batterie au lithium, sodium ou potassium; un accumulateur lithium-air ou lithium-soufre.

Selon un mode de réalisation particulier, l'électrolyte est mis en œuvre dans une batterie, en particulier une batterie au lithium.

L'invention va maintenant être décrite au moyen des exemples et figures suivants, soumis à titre illustratif et non limitatif de l'invention.
Figure 1 : Diagramme voltampérométrie cyclique (CV) à 10 mV/s du composé IA (exemple 1) en mode réduction (-0.12 V à +0.90 V vs Ag/Ag⁺).
Figure 2 : Diagramme voltampérométrie cyclique (CV) à 10 mV/s du composé IA (exemple 1) (en mode oxydation (0,00 V à -3,10 V vs Ag/Ag⁺).
Figure 3 : Spectre 1H NMR (DMSOd6, 400 MHz) du composé IB
Figure 4 : Analyse calorimétrique du composé IB par DSC.

### EXEMPLE 1

### Synthèse des composés IA et IB selon l'invention

### Etape a)

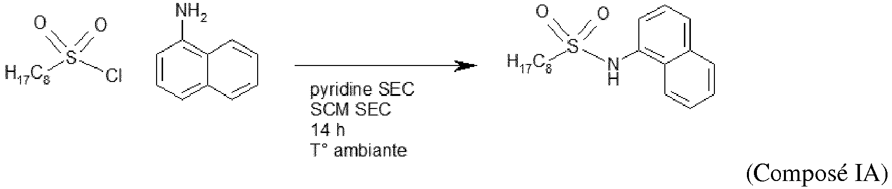

Un ballon séché au four est chargé avec du 1-aminonaphtalène (1-AN, 3,70 g, 25,9 mmol, 1,10 équivalent) et de la pyridine (4 mL). La solution légèrement pourpre et agitée est ensuite additionnée de chlorure de 1-octanesulfonyle (1-OSC, 5 g, 23,5 mmol) dans du DCM (1 mL), goutte à goutte à 0 ° C, pendant 15 minutes. La réaction est exothermique. Lors de l'ajout de l'OSC, la solution forme d'abord une robe claire, vert citron, puis devient magenta clair et brillant. La solution s'assombrit avec le temps. Après agitation pendant 18 h, la solution est acidifiée avec du HCl (2 M, 12 mL), induisant une séparation des phases. La phase organique est diluée avec du DCM (20 mL), séparée, puis extraite à nouveau avec du HCl (2 M, 12 mL). La phase aqueuse est extraite à nouveau avec du DCM (2 x 10 mL). Les phases organiques sont combinées, extraites avec de l'eau et de la saumure, puis séchées sur MgSO₄. Le solvant est éliminé sous vide pour obtenir le produit. Un produit unique est observé par TLC (1: 1 MeOH / CHCl₃ comme éluant).

Rendement = 7,07 g, 22,1 mmol, 94,2%.

La pureté de l'échantillon a été confirmée par RMN 1H (DMSO-d6, 200 MHz), δ (ppm): NH 9,75, Ar-H (1H) 8,27, Ar-H (2H) 7,94, Ar-H (2H) 7,82 , Ar-H (4H) 7,54, CH₂SO₂ 3,09, CH₂CH₂SO₂ 1,69, CH2 (10H) 1,17, CH₃ 0,83.

Le composé IA est caractérisé par voltammétrie cyclique en oxydation et en réduction.

Le voltampérogramme (CV) du composé IA (0.1 M dans l'acétonitrile) en réduction (-0.12 V to +0.90 V vs Ag/Ag⁺), a été mesuré à 10 mV/s (10 cycles) avec une électrode de travail en carbone vitreux et une contre-électrode de surface plus importante en carbone vitreux. L'électrode de référence est formée d'un fil d'argent immergé dans une solution contenant 0,01 M d'AgNO₃ et 0,1 M de tétrafluoroborate tétraéthylammonium dans de l'acétonitrile.

Le voltampérogramme (CV) du composé IA (0.1 M dans l'acétonitrile) en oxydation (0,00 V to -3,10 V vs Ag/Ag⁺), a également été mesuré à 10 mV/s (7 cycles) avec une électrode de travail en carbone vitreux et une contre-électrode de surface plus importante en carbone vitreux. L'électrode de référence est également formée d'un fil d'argent immergé dans une solution contenant 0,01 M d'AgNO₃ et 0,1 M de tétrafluoroborate tétraéthylammonium dans de l'acétonitrile.

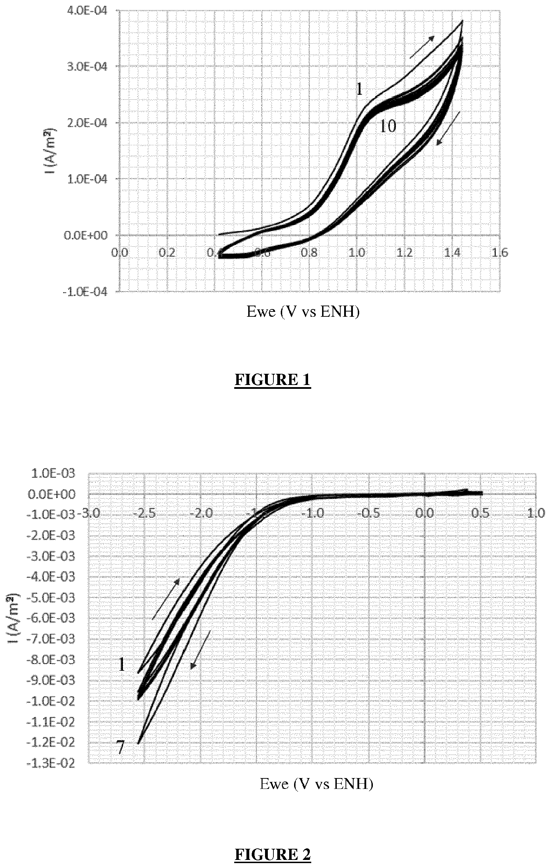

Les Figures 1 et 2 rendent respectivement compte des courbes obtenues.

Le composé I(A) est stable sur une fenêtre électrochimique de [-2.0V - 0.8V] vs Ag/Ag+. Aucune vague significative d'oxydation n'est apparente (courant < 50nA/cm²). Une courant de réduction significatif (> 1µA/cm²) est observable en dessous de -2.0V vs Ag/Ag+.

Le sel de lithium est ensuite obtenu comme suit :

### Etape b)

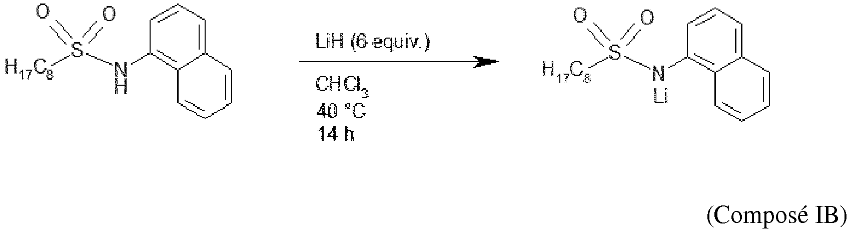

Un flacon sec est chargé avec du N-(naphtyl) octanesulfonamide 1 (0,508 g, 1,59 mmol) et du CHCl₃ (4 ml), puis chauffé à 40°C. Le LiH est ajouté lentement (75,8 mg, 9,54 mmol, 6 équivalents) à la solution sous agitation. Le ballon est scellé et chauffé sous agitation pendant une nuit. Après 18 h, du MeOH (3 mL) est ajouté goutte à goutte au mélange réactionnel. La solution est refroidie à température ambiante, concentrée sous pression réduite, puis diluée avec du propan-2-ol. La suspension résultante est filtrée sous pression réduite et le filtrat recueilli. Les solvants sont éliminés par évaporation rotative.

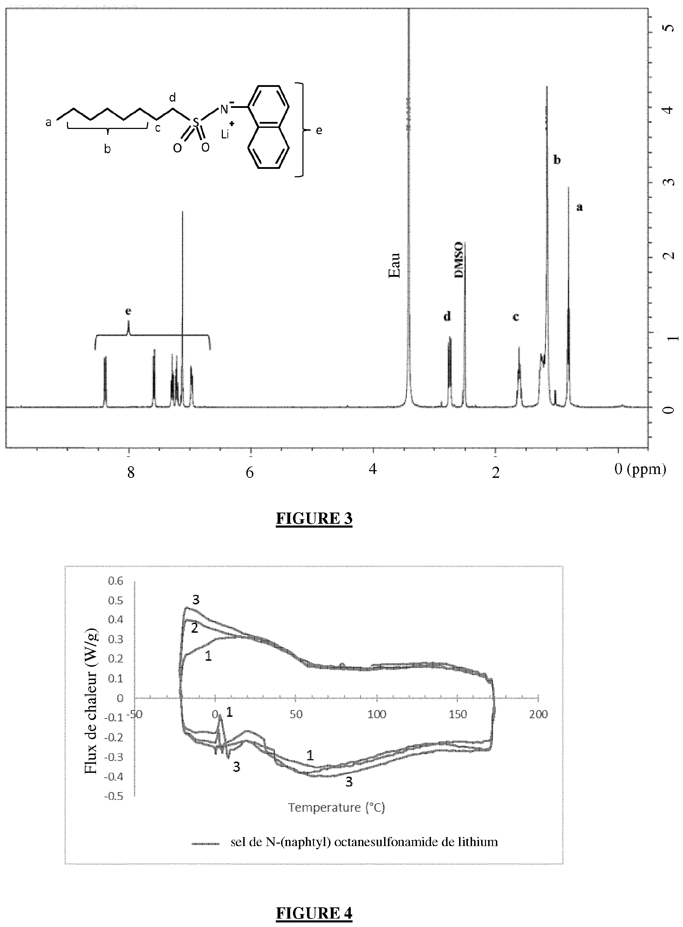

Le composé obtenu a été analysé par RMN 1H (voir figure 3). On note la disparition du proton de sulfonamide observé dans le composé de départ.

Le composé IB a été caractérisé par analyse calorimétrique différentielle à balayage (DSC) sous argon et avec une vitesse de chauffe de 5 °C/min de - 20 °C à 180 °C. Les cycles sont numérotés 1,2,3.

Les résultats sont présentés en figure 4.

Le composé IB présente une endotherme vers 20°C et un exotherme vers 50°C, signe d'une cristallisation/fusion réversible sur 3 cycles.

### EXEMPLE 2

### Synthèse du composé IC selon l'invention

### Etape a)

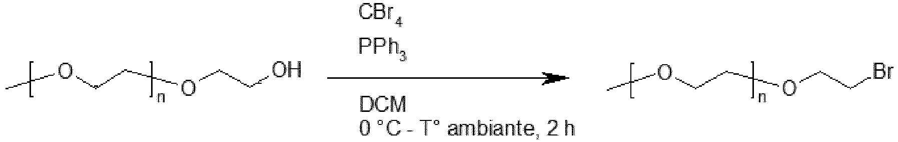

Un flacon est chargé avec une solution de méthoxy-PEO-OH (Mn = 500 g/mol, 5,00 g, 10 mmoles de groupes terminaux hydroxy) et CBr₄ (4,97 g, 15 mmoles) dans du dichlorométhane (DCM, 25 mL) et refroidi à 0°C. A cette solution incolore claire, est ajouté PPh₃ (3,93 g, 15 mmoles) sous la forme d'une poudre sèche pendant 15 minutes à 0°C. On laisse la solution se réchauffer à température ambiante et on poursuit l'agitation pendant une nuit. La solution est ensuite concentrée sous pression réduite et précipitée à partir d'heptane froid (1 x 75 mL) et de pentane (3 x 20 mL). Le solide est séché sous pression réduite, puis repris dans de l'éther diéthylique et filtré. L'éther diéthylique est éliminé sous pression réduite pour obtenir le produit méthoxy-PEO-Br (580 g / mol) sous la forme d'une huile jaune clair (4,26 g, 7,57 mmol, rendement 75,7%).

### Etape b)

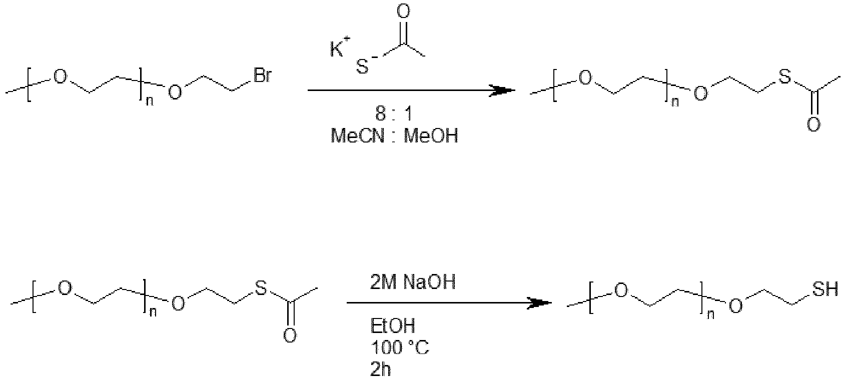

Un ballon est chargé avec du méthoxy-PEO-Br (580 g / mol, 1,00 g, 1,72 mmol) dans du MeCN (5 ml). La solution est agitée pendant 1 heure à température ambiante. Au mélange sous agitation, est ajouté du thioacétate de potassium (295 mg, 2,59 mmol, 1,5 équivalent) sous la forme d'une solution dans du MeCN (11 ml) / EtOH (2 ml). Le mélange réactionnel est agité à température ambiante pendant une nuit puis concentré sous pression réduite (109 à 115 mbar, 25 à 28°C). Il est ensuite ajouté de l'EtOH (10 mL), puis du NaOH 2 M (2 ml, 4 mmoles). Le milieu réactionnel est chauffé au reflux (100°C, 2 h) puis neutralisé par addition de 2M de HCl (2 mL, 4 mmol). Les composés volatils sont éliminés sous pression réduite. Le résidu huileux est repris dans du DCM et filtré pour obtenir le produit brut de méthoxy-PEO-SH sous la forme d'une huile.

### Etape c)

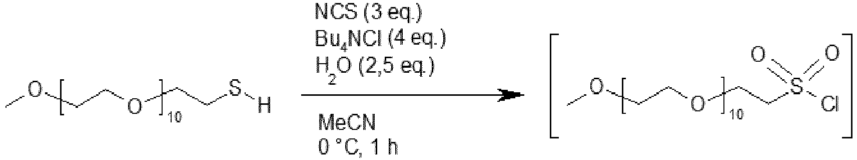

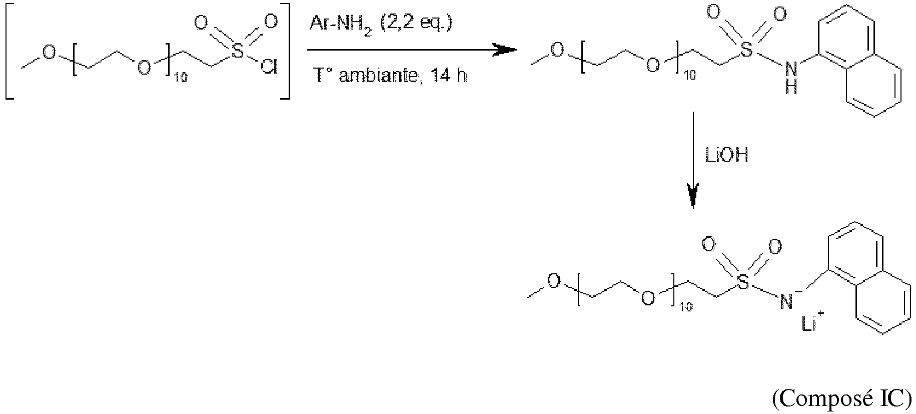

Un flacon est chargé avec du méthoxy-PEO-SH (500 mg, 516 g / mol, 0,97 mmol) dans de l'acétonitirile (MeCN, 10 ml) et de l'eau déionisée (43,6 mg, 2,42 mmol, 2,5 équivalents) et l'ensemble est refroidi à 0°C. Sont ensuite ajoutés du chlorure de tétrabutylammonium (Bu₄NCl, 1,077 g, 3,88 mmol, 4,0 équivalents) et du N-chlorosuccinimide (NCS, 388 mg, 29,07 mmol, 3,0 équivalents) et le mélange réactionnel est agité à 0°C pendant 1 heure. Du 1-amino-naphtalène (305 mg, 2,122 mmol, 2,2 équivalents) est ensuite ajouté au mélange sous agitation, qui est ensuite laissé à température ambiante. Le mélange réactionnel est agité pendant une nuit. Du MeCN (10 mL) est ajouté pour diluer le mélange. La suspension est filtrée et le précipité lavé avec du MeCN (4 x 10 mL). Les solutions sont combinées et le solvant éliminé sous pression réduite. Le produit brut est repris dans du CHCl₃ et passé sur une colonne de gel de silice. Le solvant est évaporé et le résidu repris dans de l'eau désionisée, traité avec de l'hydroxyde de lithium monohydraté jusqu'à à un virage pH-métrique puis extrait avec du CHCl₃. Les phases organiques combinées sont ensuite séchées sur K₂CO₃ et le solvant éliminé sous pression réduite pour obtenir le produit sous la forme d'une huile violette foncée.

## Revendications

1. Composé de type sulfonamide asymétrique comprenant au moins:
• un groupement polycyclique, Ar, formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ;
• une chaîne aliphatique, linéaire ou ramifiée, saturée ou insaturée, ladite chaîne étant éventuellement interrompue par un ou plusieurs hétéroatomes, par un ou plusieurs métalloïdes, éventuellement substituée par un ou plusieurs atomes de fluor, et/ou contenant un ou plusieurs groupes choisis dans le groupe constitué des groupes hydroxyle, -NH₂ et oxo, ladite chaîne aliphatique comprenant un enchaînement linéaire d'au moins six liaisons covalentes ;
• ledit groupement Ar et ladite chaine aliphatique étant liés de manière covalente *via* un espaceur figuré par un motif sulfonamide -SO₂-NH- ou sa forme anionique -SO₂-N⁻-, ledit sulfonamide étant lié audit groupement polycyclique Ar *via* son atome d'azote et à ladite chaîne aliphatique *via* son atome de soufre ; et, le cas échéant
• un contre-cation de la forme anionique du motif sulfonamide, choisi parmi les métaux alcalins et le proton H⁺ ou H₃O⁺.

2. Composé selon la revendication précédente se présentant sous la forme d'un sel possédant, à titre d'espaceur, la forme anionique dudit sulfonamide -SO₂-N⁻-, et un contre-cation, de préférence alcalin, et en particulier un atome de lithium.

3. Composé selon l'une quelconque des revendications précédentes dans lequel le lien covalent entre l'atome d'azote et ledit groupement polycyclique est établi *via* une structure aromatique formant tout ou partie dudit groupement polycyclique.

4. Composé selon l'une quelconque des revendications précédentes dans lequel ledit groupement polycyclique Ar est sous une forme condensée aromatique.

5. Composé selon l'une quelconque des revendications précédentes, dans lequel ledit groupement polycyclique Ar présente l'un des squelettes polycycliques suivants ou dérive de l'un des squelettes polycycliques suivants : 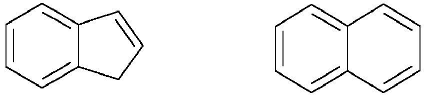 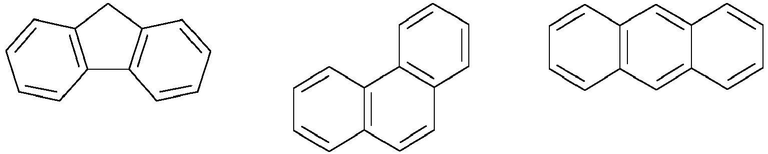 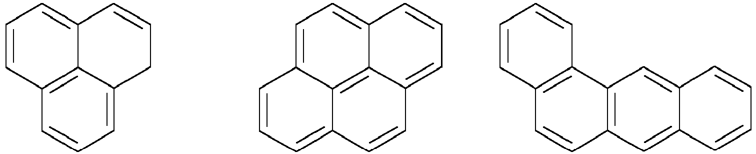 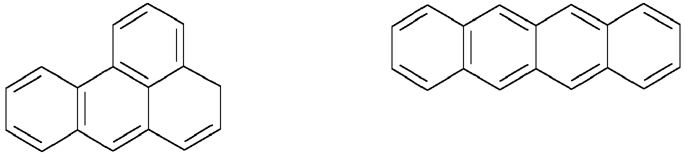 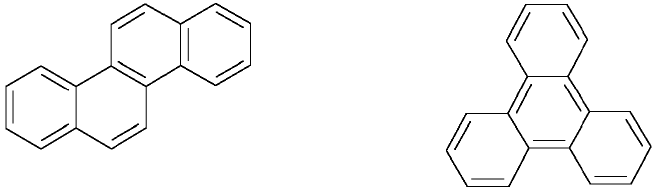 ledit groupement polycyclique Ar étant en particulier un radical dérivant d'un squelette bicyclique aromatique et plus particulièrement un radical du naphtalène.

6. Composé selon l'une quelconque des revendications précédentes dans lequel la chaîne aliphatique est une chaîne alkyle linéaire comprenant de 6 à 18 atomes de carbone, de préférence de 6 à 12 atomes de carbone, éventuellement fluorée, semifluorée, voire perfluorée, et/ou éventuellement substituée par au moins un, voire plusieurs groupe(s) hydroxyle(s), et éventuellement entrecoupée par un ou plusieurs atome(s) d'oxygène.

7. Composé selon l'une quelconque des revendications précédentes dans lequel la chaîne aliphatique possède une structure d'oligomère, d'homopolymère ou de copolymère, ladite chaîne aliphatique possédant en particulier un enchainement d'au moins 5, voire plus, motifs oxyde d'éthylène.

8. Composé selon l'une quelconque des revendications précédentes répondant à la formule générale : 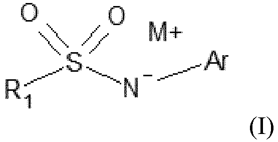 dans laquelle
- Ar est un groupement polycyclique totalement aromatique tel que défini en revendications 1 à 5 et de préférence représente un radical naphtyle,
- R₁ représente une chaîne aliphatique telle que définie en revendication 1 et 6 à 7 et
- M⁺ est un contre-cation choisi dans le groupe des métaux alcalins et en particulier est le Li⁺.

9. Composé selon l'une quelconque des revendications précédentes de formule 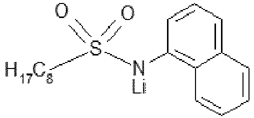 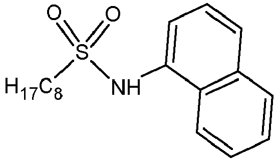 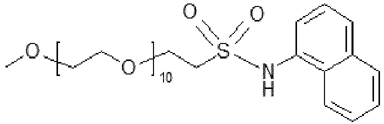 ou

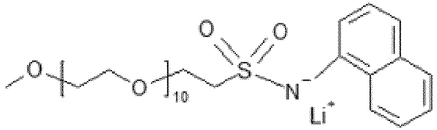

10. Utilisation à titre d'électrolyte de la forme anionique d'un composé de type sulfonamide asymétrique comprenant au moins :
• un groupement polycyclique, Ar, formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ;
• une chaîne aliphatique, linéaire ou ramifiée, saturée ou insaturée, ladite chaîne étant éventuellement interrompue par un ou plusieurs hétéroatomes, par un ou plusieurs métalloïdes, éventuellement substituée par un ou plusieurs atomes de fluor, et/ou contenant un ou plusieurs groupes choisis dans le groupe constitué des groupes hydroxyle, -NH₂ et oxo ;
• ledit groupement Ar et ladite chaine aliphatique étant liés de manière covalente *via* un espaceur figuré par un motif sulfonamide -SO₂-NH- ou sa forme anionique -SO₂-N⁻- ; et, le cas échéant
• un contre-cation de la forme anionique du motif sulfonamide, choisi parmi les métaux alcalins et le proton H⁺ ou H₃O⁺.

11. Utilisation selon la revendication précédente, ledit composé étant tel que défini selon l'une quelconque des revendications 1 à 9.

12. Electrolyte, notamment pour systèmes électrochimiques, formé en tout ou partie par la forme anionique d'un composé selon l'une quelconque des revendications 1 à 9.

13. Système électrochimique comprenant un électrolyte formé en tout ou partie par la forme anionique d'un composé de type sulfonamide asymétrique comprenant au moins :
• un groupement polycyclique, Ar, formé de deux à six cycles dont l'un au moins est aromatique, lesdits cycles comprenant, indépendamment les uns des autres, de 4 à 6 chaînons, ledit groupement polycyclique pouvant inclure jusqu'à 18 hétéroatomes, en particulier choisis dans le groupe constitué de S, N et O ;
• une chaîne aliphatique, linéaire ou ramifiée, saturée ou insaturée, ladite chaîne étant éventuellement interrompue par un ou plusieurs hétéroatomes, par un ou plusieurs métalloïdes, éventuellement substituée par un ou plusieurs atomes de fluor, et/ou contenant un ou plusieurs groupes choisis dans le groupe constitué des groupes hydroxyle, -NH₂ et oxo ;
• ledit groupement Ar et ladite chaine aliphatique étant liés de manière covalente *via* un espaceur figuré par un motif sulfonamide -SO₂-NH- ou sa forme anionique -SO₂-N⁻- ; et, le cas échéant
• un contre-cation de la forme anionique du motif sulfonamide, choisi parmi les métaux alcalins et le proton H⁺ ou H₃O⁺.

14. Système électrochimique selon la revendication précédente, dans lequel ledit composé est tel que défini selon l'une quelconque des revendications 1 à 9.

15. Système électrochimique selon la revendication 13 ou 14, **caractérisé en ce qu'**il s'agit d'un système générateur, convertisseur ou de stockage électrochimique, en particulier une pile à combustible à membrane échangeuse de protons (PEMFC) ou batterie redox-flow; une batterie primaire ou secondaire, par exemple une batterie au lithium, sodium ou potassium; un accumulateur lithium-air ou lithium-soufre.

## Patentansprüche

1. Asymmetrische Sulfonamidverbindung, die zumindest Folgendes umfasst:
• eine polycyclische Gruppe Ar, die aus zwei bis sechs Ringen besteht, von denen mindestens einer aromatisch ist, wobei die Ringe unabhängig voneinander 4 bis 6 Glieder umfassen, die polycyclische Gruppe bis zu 18 Heteroatome einschließen kann, die insbesondere ausgewählt sind aus der Gruppe bestehend aus S, N und O;
• eine lineare oder verzweigte, gesättigte oder ungesättigte aliphatische Kette, wobei die Kette gegebenenfalls durch ein oder mehrere Heteroatome, durch ein oder mehrere Halbmetalle unterbrochen ist, gegebenenfalls durch ein oder mehrere Fluoratome substituiert ist und/oder eine oder mehrere Gruppen enthält, die ausgewählt sind aus der Gruppe bestehend aus Hydroxyl-, -NH₂- und Oxogruppen, wobei die aliphatische Kette eine lineare Abfolge von mindestens sechs kovalenten Bindungen umfasst;
• wobei die Gruppe Ar und die aliphatische Kette über einen Spacer kovalent verbunden sind, der durch eine Sulfonamideinheit -SO₂-NH- oder ihre anionische Form - SO₂-N⁻- veranschaulicht wird, wobei das Sulfonamid über sein Stickstoffatom an der polycyclischen Gruppe Ar und über sein Schwefelatom an der aliphatischen Kette gebunden ist; und, falls erforderlich,
• ein Gegenkation der anionischen Form der Sulfonamideinheit, das aus Alkalimetallen und dem Proton H⁺ oder H₃O⁺ ausgewählt ist.

2. Verbindung nach dem vorhergehenden Anspruch, die in Form eines Salzes vorliegt, das als Spacer die anionische Form des Sulfonamids -SO₂-N⁻- und ein Gegenkation, vorzugsweise alkalisch und insbesondere ein Lithiumatom, aufweist.

3. Verbindung nach einem der vorhergehenden Ansprüche, wobei die kovalente Bindung zwischen dem Stickstoffatom und der polycyclischen Gruppe über eine aromatische Struktur erfolgt, welche die gesamte oder einen Teil der polycyclischen Gruppe darstellt.

4. Verbindung nach einem der vorhergehenden Ansprüche, wobei die polycyclische Gruppe Ar in einer kondensierten aromatischen Form vorliegt.

5. Verbindung nach einem der vorhergehenden Ansprüche, wobei die polycyclische Gruppe Ar eines der folgenden polycyclischen Gerüste aufweist oder von einem der folgenden polycyclischen Gerüste abgeleitet ist: 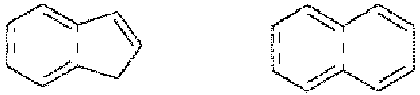 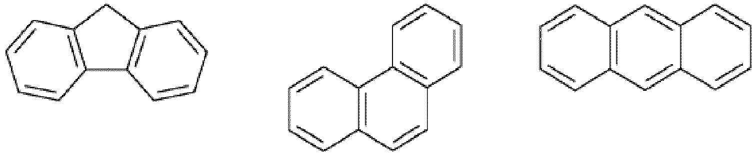 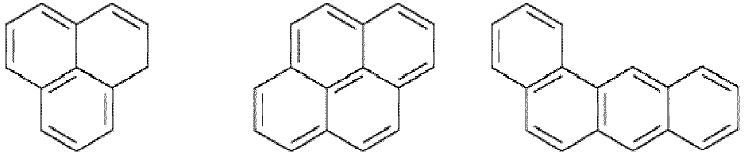 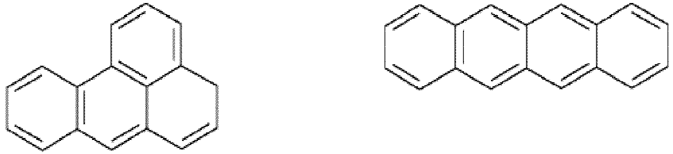 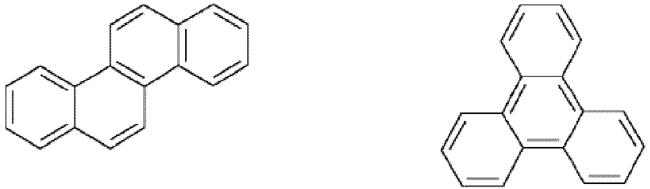 wobei die polycyclische Gruppe Ar insbesondere ein Rest ist, der von einem aromatischen bicyclischen Gerüst abgleitet und noch mehr bevorzugt ein Naphthalinrest ist.

6. Verbindung nach einem der vorhergehenden Ansprüche, wobei die aliphatische Kette eine lineare Alkylkette ist, die 6 bis 18 Kohlenstoffatome, vorzugsweise 6 bis 12 Kohlenstoffatome umfasst, die gegebenenfalls fluoriert, semifluoriert oder sogar perfluoriert und/oder gegebenenfalls durch mindestens ein oder sogar mehrere Hydroxylgruppen substituiert und gegebenenfalls durch ein oder mehrere Sauerstoffatome unterbrochen sind.

7. Verbindung nach einem der vorhergehenden Ansprüche, wobei die aliphatische Kette eine Oligomer-, Homopolymer-oder Copolymerstruktur aufweist, die aliphatische Kette insbesondere eine Abfolge von mindestens 5 oder sogar mehr Ethylenoxideinheiten aufweist.

8. Verbindung nach einem der vorhergehenden Ansprüche mit der allgemeinen Formel: 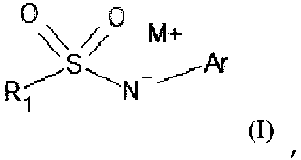 wobei
- Ar eine vollständig aromatische polycyclische Gruppe gemäß der Definition in den Ansprüchen 1 bis 5 ist und vorzugsweise für einen Naphthylrest steht,
- R₁ für eine aliphatische Kette gemäß der Definition in Anspruch 1 und 6 bis 7 steht und
- M⁺ ein Gegenkation ist, das aus der Gruppe der Alkalimetalle ausgewählt und insbesondere Li⁺ ist.

9. Verbindung nach einem der vorhergehenden Ansprüche mit der Formel 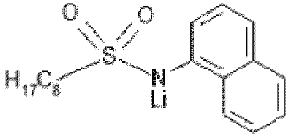 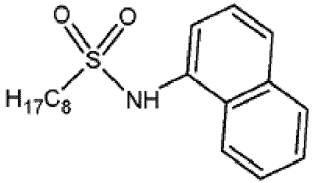 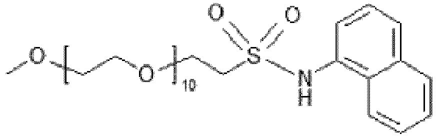 oder

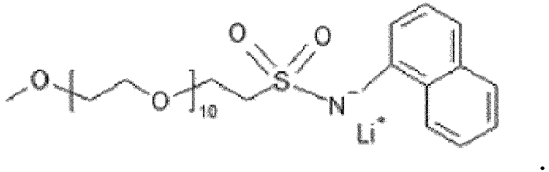

10. Verwendung als Elektrolyt der anionischen Form einer asymmetrischen Sulfonamidverbindung, die zumindest Folgendes umfasst:
• eine polycyclische Gruppe Ar, die aus zwei bis sechs Ringen besteht, von denen mindestens einer aromatisch ist, wobei die Ringe unabhängig voneinander 4 bis 6 Glieder umfassen, die polycyclische Gruppe bis zu 18 Heteroatome einschließen kann, die insbesondere ausgewählt sind aus der Gruppe bestehend aus S, N und O;
• eine lineare oder verzweigte, gesättigte oder ungesättigte aliphatische Kette, wobei die Kette gegebenenfalls durch ein oder mehrere Heteroatome, durch ein oder mehrere Halbmetalle unterbrochen ist, gegebenenfalls durch ein oder mehrere Fluoratome substituiert ist und/oder eine oder mehrere Gruppen enthält, die ausgewählt sind aus der Gruppe bestehend aus Hydroxyl-, -NH₂- und Oxogruppen;
• wobei die Gruppe Ar und die aliphatische Kette über einen Spacer kovalent verbunden sind, der durch eine Sulfonamideinheit -SO₂-NH- oder ihre anionische Form - SO₂-N⁻- veranschaulicht wird; und, falls erforderlich,
• ein Gegenkation der anionischen Form der Sulfonamideinheit, das aus Alkalimetallen und dem Proton H⁺ oder H₃O⁺ ausgewählt ist.

11. Verwendung nach dem vorhergehenden Anspruch, wobei die Verbindung in einem der Ansprüche 1 bis 9 definiert ist.

12. Elektrolyt, insbesondere für elektrochemische Systeme, der ganz oder teilweise aus der anionischen Form einer Verbindung gemäß einem der Ansprüche 1 bis 9 besteht.

13. Elektrochemisches System, das einen Elektrolyten umfasst, der ganz oder teilweise aus der anionischen Form einer asymmetrischen Sulfonamidverbindung besteht, die zumindest Folgendes umfasst:
• eine polycyclische Gruppe Ar, die aus zwei bis sechs Ringen besteht, von denen mindestens einer aromatisch ist, wobei die Ringe unabhängig voneinander 4 bis 6 Glieder umfassen, die polycyclische Gruppe bis zu 18 Heteroatome einschließen kann, die insbesondere ausgewählt sind aus der Gruppe bestehend aus S, N und O;
• eine lineare oder verzweigte, gesättigte oder ungesättigte aliphatische Kette, wobei die Kette gegebenenfalls durch ein oder mehrere Heteroatome, durch ein oder mehrere Halbmetalle unterbrochen ist, gegebenenfalls durch ein oder mehrere Fluoratome substituiert ist und/oder eine oder mehrere Gruppen enthält, die ausgewählt sind aus der Gruppe bestehend aus Hydroxyl-, -NH₂- und Oxogruppen;
• wobei die Gruppe Ar und die aliphatische Kette über einen Spacer kovalent verbunden sind, der durch eine Sulfonamideinheit -SO₂-NH- oder ihre anionische Form - SO₂-N⁻- veranschaulicht wird; und, falls erforderlich,
• ein Gegenkation der anionischen Form der Sulfonamideinheit, das aus Alkalimetallen und dem Proton H⁺ oder H₃O⁺ ausgewählt ist.

14. Elektrochemisches System nach dem vorhergehenden Anspruch, wobei die Verbindung in einem der Ansprüche 1 bis 9 definiert ist.

15. Elektrochemisches System nach Anspruch 13 oder 14, **dadurch gekennzeichnet, dass** es sich um ein elektrochemisches Erzeugungs-, Umwandlungs- oder Speichersystem, insbesondere eine Protonenaustauschmembran-Brennstoffzelle (PEMFC) oder eine Redox-Flow-Batterie; eine Primär- oder Sekundärbatterie, beispielsweise eine Lithium-, Natrium-oder Kaliumbatterie; einen Lithium-Luft- oder einen Lithium-Schwefel-Akkumulator handelt.

## Claims

1. An asymmetric sulfonamide compound comprising at least:
• a polycyclic group, Ar, consisting of two to six rings, at least one of which is aromatic, said rings each independently having from 4 to 6 members, said polycyclic group being able to include up to 18 heteroatoms, in particular selected from the group consisting of S, N and O;
• a linear or branched, saturated or unsaturated aliphatic chain, said chain being optionally interrupted by one or more heteroatoms, by one or more metalloids, optionally substituted by one or more fluorine atoms, and/or containing one or more groups selected from the group consisting of hydroxyl, -NH₂ and oxo groups, said aliphatic chain comprising a linear sequence of at least six covalent bonds;
• said group Ar and said aliphatic chain being covalently linked via a spacer represented by a sulfonamide unit -SO₂-NH- or its anionic form -SO₂-N⁻-, said sulfonamide being linked to said polycyclic group Ar via its nitrogen atom and to said aliphatic chain via its sulfur atom; and, if need be
• a counter cation for the anionic form of the sulfonamide unit, selected from the alkali metals and the proton H⁺ or H₃O⁺.

2. The compound as claimed in the preceding claim in the form of a salt having, as spacer, the anionic form of said sulfonamide -SO₂-N⁻-, and a counter cation, preferably alkaline, and in particular a lithium atom.

3. The compound as claimed in any one of the preceding claims, wherein the covalent bond between the nitrogen atom and said polycyclic group is established via an aromatic structure forming all or part of said polycyclic group.

4. The compound as claimed in any one of the preceding claims, wherein said polycyclic group Ar is in a condensed aromatic form.

5. The compound as claimed in any one of the preceding claims, wherein said polycyclic group Ar has one of the following polycyclic skeletons or is derived from one of the following polycyclic skeletons: 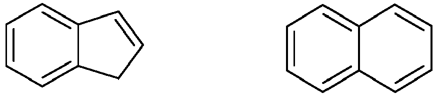 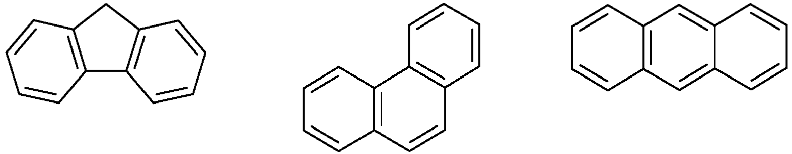 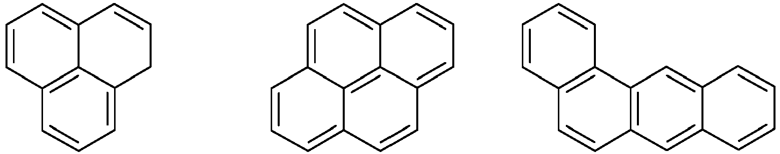 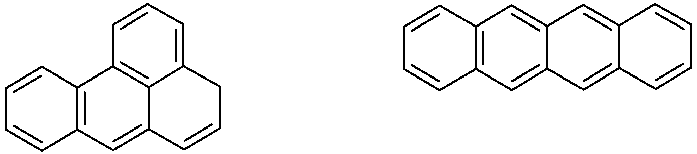 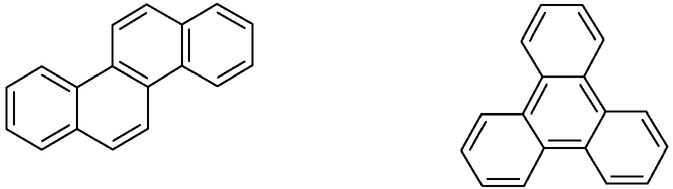 said polycyclic group Ar being in particular a radical derived from an aromatic bicyclic skeleton and more particularly a naphthalene radical.

6. The compound as claimed in any one of the preceding claims, wherein the aliphatic chain is a linear alkyl chain including from 6 to 18 carbon atoms, preferably from 6 to 12 carbon atoms, optionally fluorinated, semifluorinated, or even perfluorinated, and/or optionally substituted by at least one or more hydroxyl group(s) and optionally intersected by one or more oxygen atom(s).

7. The compound as claimed in any one of the preceding claims, wherein the aliphatic chain has an oligomeric, homopolymeric or copolymeric structure, said aliphatic chain having in particular a chain of at least 5 or more ethylene oxide units.

8. The compound as claimed in any one of the preceding claims having the general formula: 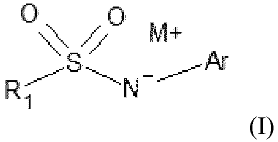 wherein
- Ar is a fully aromatic polycyclic group as defined in claims 1 to 5 and preferably represents a naphthyl radical,
- R₁ represents an aliphatic chain as defined in claim 1 and 6 to 7 and
- M⁺ is a counter cation selected from the group of alkali metals and in particular is Li⁺.

9. The compound as claimed in any one of the preceding claims of formula 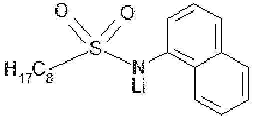 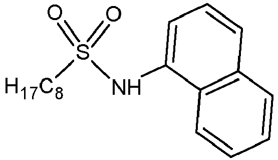 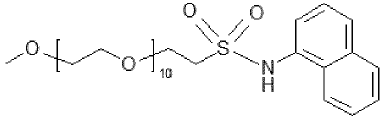 or

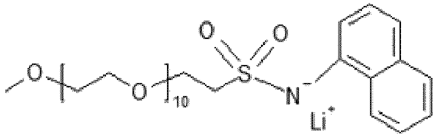

10. The use as electrolyte of the anionic form of an asymmetric sulfonamide compound comprising at least:
• a polycyclic group, Ar, consisting of two to six rings, at least one of which is aromatic, said rings each independently having from 4 to 6 members, said polycyclic group being able to include up to 18 heteroatoms, in particular selected from the group consisting of S, N and O;
• a linear or branched, saturated or unsaturated aliphatic chain, said chain being optionally interrupted by one or more heteroatoms, by one or more metalloids, optionally substituted by one or more fluorine atoms, and/or containing one or more groups selected from the group consisting of hydroxyl, -NH₂ and oxo groups;
• said group Ar and said aliphatic chain being covalently linked via a spacer represented by a sulfonamide unit -SO₂-NH- or its anionic form -SO₂-N⁻-; and, if need be
• a counter cation for the anionic form of the sulfonamide unit, selected from the alkali metals and the proton H⁺ or H₃O⁺.

11. The use as claimed in the preceding claim, said compound being as defined according to any one of claims 1 to 9.

12. An electrolyte, in particular for electrochemical systems, formed wholly or partially by the anionic form of a compound as claimed in any one of claims 1 to 9.

13. An electrochemical system comprising an electrolyte formed in whole or in part by the anionic form of an asymmetric sulfonamide compound comprising at least:
• a polycyclic group, Ar, consisting of two to six rings, at least one of which is aromatic, said rings each independently having from 4 to 6 members, said polycyclic group being able to include up to 18 heteroatoms, in particular selected from the group consisting of S, N and O;
• a linear or branched, saturated or unsaturated aliphatic chain, said chain being optionally interrupted by one or more heteroatoms, by one or more metalloids, optionally substituted by one or more fluorine atoms, and/or containing one or more groups selected from the group consisting of hydroxyl, -NH₂ and oxo groups;
• said group Ar and said aliphatic chain being covalently linked via a spacer represented by a sulfonamide unit -SO₂-NH- or its anionic form -SO₂-N⁻-; and, if need be
• a counter cation for the anionic form of the sulfonamide unit, selected from the alkali metals and the proton H⁺ or H₃O⁺.

14. The electrochemical system as claimed in the preceding claim, wherein said compound is as defined according to any one of claims 1 to 9.

15. The electrochemical system as claimed in claim 13 or 14, **characterized in that** it is an electrochemical generator, converter or storage system, in particular a proton exchange membrane fuel cell (PEMFC) or redox flow battery; a primary or secondary battery, for example a lithium, sodium or potassium battery; a lithium-air or lithium-sulfur battery.
